# EUROPÄISCHE PATENTSCHRIFT

(11) **EP 2 456 781 B1**
(45) Veröffentlichungstag und Bekanntmachung des Hinweises auf die Patenterteilung: **21.06.2017**
(21) Anmeldenummer: 10730384.4
(22) Anmeldetag: 07.07.2010
(51) Int. Cl.: C07J 1/00, A61K 31/58, C07J 43/00

(54) **17-HYDROXY-17-PENTAFLUORETHYL-ESTRA-4,9 (10)-DIEN-11-ACYLOXYALKYLENPHENYL-DERIVATE, VERFAHREN ZU IHRER HERSTELLUNG UND IHRE VERWENDUNG ZUR BEHANDLUNG VON KRANKHEITEN**
17-HYDROXY-17-PENTAFLUORETHYL-ESTRA-4,9(10)-DIEN-11-ACYLOXYALKYLENE PHENYL DERIVATIVES, METHODS FOR THE PRODUCTION THEREOF AND USE THEREOF FOR TREATING DISEASES
DÉRIVÉS 17-HYDROXY-17-PENTAFLUOROÉTHYL-ESTRA-4,9(10)-DIÈNE-11-ACYLOXYALCÉNYLPHÉNYLE, PROCÉDÉS DE PRÉPARATION ET UTILISATION POUR LE TRAITEMENT DE MALADIES

(30) Priorität: 20.07.2009 DE 102009034368
(43) Veröffentlichungstag der Anmeldung: 30.05.2012
(73) Patentinhaber: Bayer Intellectual Property GmbH, 40789 Monheim (DE)
(72) Erfinder: KLAR, Ulrich, 13503 Berlin (DE); SCHWEDE, Wolfgang, 16548 Glienicke (DE); MÖLLER, Carsten, 10115 Berlin (DE); ROTGERI, Andrea, 13503 Berlin (DE); KRENZ, Ursula, 42799 Leichlingen (DE)
(74) Vertreter: BIP Patents
(86) Internationale Anmeldenummer: PCT/EP2010/004148
(87) Internationale Veröffentlichungsnummer: WO 2011/009530

(56) Entgegenhaltungen:
- WO-A1-98/34947

## Beschreibung

Die Erfindung betrifft 17-Hydroxy-13-methyl-17-pentafluorethyl-11-acyloxyalkylenphenyl-dodecahydro-cyclopenta[a]phenanthren-3-on-Derivate der Formel I mit Progesteron antagonisierender Wirkung und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden. Zur Behandlung von Uterusfibroiden und der Endometriose können die erfindungsgemäßen Verbindungen auch sequentiell in Kombination mit Gestagenen verabreicht werden. In einem solchen Behandlungsregime könnten die erfindungsgemäßen Verbindungen über einen Zeitraum von 1 - 6 Monaten gegeben werden, gefolgt von einer Behandlungspause oder einer sequentiellen Behandlung mit einem Gestagen über einen Zeitraum von 2 - 6 Wochen oder gefolgt von der Behandlung mit einem oralen Kontrazeptivum (OC-Kombinationen) über den gleichen Zeitraum.

Die Wirksamkeit der erfindungsgemäßen Verbindungen als Progesteronrezeptorantagonist wurde in vitro in Transaktivierungstests gezeigt.

Verbindungen mit antagonistischer Wirkung am Progesteronrezeptor (kompetitive Progesteronrezeptorantagonisten) sind erstmals 1982 erwähnt (RU 486; EP57115) und seither zahlreich beschrieben worden. Progesteronrezeptorantagonisten mit fluorierter 17α-Seitenkette wurden in WO 98/34947 und Fuhrmann et al., J. Med. Chem. 43, 5010 - 5016 (2000) veröffentlicht.

Die in WO 98/34947 beschriebenen Verbindungen mit fluorierter 17α-Seitenkette weisen im allgemeinen eine sehr starke antagonistische Aktivität am Progesteronrezeptor auf. Sehr potente und daher in WO 98/34947 bevorzugte Verbindungen sind 11β-(4-Acetylphenyl)-20,20,21,21,21-pentafluor-17-hydroxy-19-nor-17α-pregna-4,9-dien-3-on, 11β-(4-Acetylphenyl)-20,20,21,21,21-pentafluor-17-hydroxy-19-nor-17α-pregna-4-en-3-on und 6'-Acetyl-9,11β-dihydro-17β-hydroxy-17α-(1,1,2,2,2-pentafluorethyl)-4'H-naphth [3',2',1':10,9,11]ester-4-en-3-on. Diese Verbindungen werden in vivo in erheblichem Maße in verschiedene Metabolite überführt, die zum Teil starke, zum Teil geringere pharmakologische Aktivität aufweisen. Der Metabolismus tritt überwiegend am 4-Substituenten des 11-Acetyl-Phenylsubstituenten auf. In WO2008/058767 werden Verbindungen beschrieben, die zumindest zum Teil Metabolite der in WO 98/34947 beschriebenen Verbindungen sind.

Ein Teil der erfindungsgemäßen Verbindungen erwies sich unter physiologischen Bedingungen als stabil und dennoch hoch aktiv, so dass auch neue kompetitive Progesteronrezeptorantagonisten zur Verfügung gestellt werden konnten.
Die vorliegende Erfindung betrifft 17-Hydroxy-13-methyl-17-pentafluorethyl-11-acyloxyalkylenphenyl-dodecahydro-cyclopenta[a]phenanthren-3-on-Derivate mit der allgemeinen chemischen Formel I: 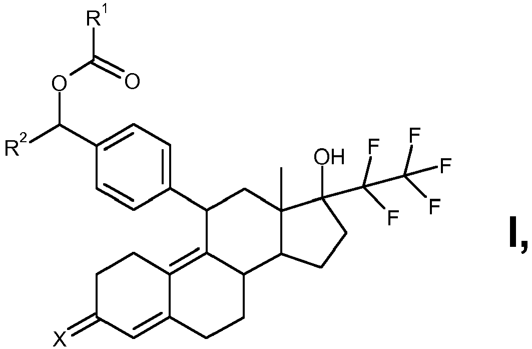 in welcher
- X: Sauerstoff, eine Gruppe NOR³ oder =NNHSO₂R³,
- R¹: C₁-C₁₀-Alkyl, (CH₂)ₙ-Y oder CHR⁴NR⁵PG,
- R²: Wasserstoff oder C₁-C₁₀-Alkyl,
- R³: Wasserstoff, C₁-C₁₀-Alkyl, Aryl oder C₇-C₂₀-Aralkyl,
- n: 1 bis 10,
- Y: Wasserstoff, Aryl, Heteroaryl
- R⁴, R⁵: unabhängig voneinander Wasserstoff, C₁-C₁₀-Alkyl, C₇-C₁₀-Aralkyl, oder gemeinsam eine (CH₂)ₘ- oder eine CH₂CHOHCH₂-Gruppe,
- m: 3 oder 4 und
- PG: Wasserstoff oder eine Aminoschutzgruppe
bedeutet und ihre Salze, Solvate oder Solvate der Salze, einschließlich aller Kristallmodifikationen, die α-, β- oder γ-Cyclodextrinclathrate, sowie die mit Liposomen verkapselten Verbindungen bedeuten.

Die erfindungsgemäßen Verbindungen der allgemeinen Formel I können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.
Jeder der genannten Substituenten am Steroid-Grundgerüst kann sowohl in einer α-als auch in einer β-Stellung vorliegen. Außerdem können auch die Substituenten am Steroid-Grundgerüst, die eine Doppelbindung enthalten und in denen die Doppelbindung an jedem Atom mindestens einen Substituenten, der nicht Wasserstoff ist, trägt, sowohl E- als auch Z-konfiguriert vorliegen.
Sofern die erfindungsgemäßen Verbindungen in tautomeren Formen vorkommen können, umfasst die vorliegende Erfindung sämtliche tautomere Formen.
Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.
Physiologisch unbedenkliche Salze der erfindungsgemäßen Verbindungen umfassen - wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure oder Benzoesäure.
Physiologisch unbedenkliche Salze der erfindungsgemäßen Verbindungen umfassen - wenn eine saure Funktion enthaltend ist - Alkalimetallsalze, Erdalkalimetallsalze oder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methyl-aminomethan oder 1-Amino-2,3,4-butantriol.
Als Solvate werden im Rahmen der Erfindung solche Formen der erfindungsgemäßen Verbindungen bezeichnet, welche in festem oder flüssigem Zustand eine Adduktbildung mit Lösungsmittelmolekülen zeigen. Das Lösungsmittel kann dabei in einem stöchiometrischen oder auch unstöchiometrischen Verhältnis vorliegen. Bei stöchiometrischen Solvaten spricht man auch von Hemi-, (Semi-), Mono-, Sesqui-, Di-, Tri-, Tetra-, Penta-, usw. Solvaten. Hydrate sind eine spezielle Form der Solvate, bei denen die Koordination mit Wasser erfolgt.
Im Rahmen der vorliegenden Erfindung haben die Substituenten, soweit nicht anders spezifiziert, die folgende Bedeutung:
Alkyl steht für gerad- oder verzweigtkettige Alkylgruppen mit 1-6 Kohlenstoffatomen, wie beispielsweise Methyl, Ethyl, Propyl, Isopropyl, n-Butyl, sec.-Butyl, Isobutyl, tert.-Butyl, n-Pentyl, Isopentyl, Neopentyl Hexyl, Heptyl, Octyl, Nonyl und Decyl .
Aryl steht für einen mono- bis tricyclischen aromatischen, substituierten oder unsubstituierten carbocyclischen Rest, wie zum Beispiel Phenyl, Naphthyl, die einfach oder mehrfach mit Halogen (F, Cl, Br, I), OH, O-Alkyl, CO₂H, CO₂-Alkyl, NH₂, NH(C₁-C₁₀-Alkyl), N(C₁-C₁₀-Alkyl)₂, insbesondere N(CH₃)₂, NO₂, N₃, CN, C₁-C₁₀-Alkyl, C₂-C₁₀-Alkylen, C₂-C₁₀-Alkinyl, C₁-C₁₀-Perfluor-Alkyl, C₁-C₁₀-Acyl, C₁-C₁₀-Acyloxy-Gruppen, substituiert sein können.
Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Benzofuranyl, Benzothiophenyl, Chinolinyl, Furyl, Imidazolyl, Indazolyl, Indolyl, Isochinolinyl Oxazolyl, Pyridazinyl, Pyridyl, Pyrimidyl, Pyrrolyl, Thiazolyl, Thienyl, Pyrazolyl, Isoxazolyl, Pyrazinyl, Chinolyl oder Tetrazolyl, die einfach mit C₁-C₄-Alkyl substituiert sein können.
Aralkyl steht für Aralkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Aralkylreste kommen beispielsweise Benzyl, Phenylethyl, Naphthylmethyl, Naphthylethyl, Furylmethyl, Thienylethyl, Pyridylpropyl in Betracht. Die Ringe können einfach oder mehrfach durch Halogen, OH, O-Alkyl, CO₂H, CO₂-Alkyl, NH₂, NH(C₁-C₁₀-Alkyl), N(C₁-C₁₀-Alkyl)₂, NO₂, N₃, CN, C₁-C₂₀-Alkyl, C₁-C₁₀-Perfluor-Alkyl, C₁-C₂₀-Acyl, C₁-C₂₀-Acyloxy-Gruppen substituiert sein.
Aminoschutzgruppen sind gängige Gruppen zum Schutz von Aminofunktionen, z.B. tert.-Butoxycarbonyl (t-BOC) oder Allyloxycarbonyl
Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.
Bevorzugt sind Verbindungen der Formel (I), in welcher R¹ C₁C₁₀-Alkyl, bevorzugt C₁-C₆-Alkyl, besonders bevorzugt C₁-C₅-Alkyl, ganz besonders bevorzugt -Methyl,-Isopropyl, -Isobutyl und -Neopentyl bedeutet und ihre Salze, Solvate oder Solvate der Salze, die α-, β- oder γ-Cyclodextrinclathrate, sowie die mit Liposomen verkapselten Verbindungen bedeuten.

Ebenfalls bevorzugt sind Verbindungen der Formel (I), in welcher R¹ (CH₂)ₙ-Y mit n = 1 - 10 und Y ein aromatischer, mono- oder bicyclischer Rest mit in der Regel 5 bis 10 Ringatomen mit bis zu 5 Heteroatomen aus der Reihe S, O und N, bevorzugt (CH₂)ₙ-Y mit n = 1 - 5 und Y ein aromatischer, mono- oder bicyclischer Rest mit in der Regel 5 bis 9 Ringatomen mit bis zu 4 Heteroatomen aus der Reihe S, O und N, besonders bevorzugt n = 1 - 3 und Y = Imidazolyl, Thiazolyl oder Pyridyl bedeutet und ihre Salze, Solvate oder Solvate der Salze, die α-, β- oder γ-Cyclodextrinclathrate, sowie die mit Liposomen verkapselten Verbindungen bedeuten.

Bevorzugt sind außerdem Verbindungen der Formel (I), in welcher R¹ ein Aminosäurerest CHR⁴NR⁵PG mit R⁴ = Wasserstoff, C₁-C₅-Alkyl, C₇-C₁₀-Aralkyl, oder gemeinsam mit R⁵ eine, gegebenenfalls durch Hydroxyl substituierte Propylen- oder Butylengruppe, R⁵ = Wasserstoff und PG gleich C₁-C₅-Acyl oder C₁-C₅-Alkyloxycarbonyl, C₃-C₅-Alkenyloxycarbonyl, bevorzugt R⁴ = Wasserstoff, C₁-C₄-Alkyl, C₇-C₈-Aralkyl, oder gemeinsam mit R⁵ eine, gegebenenfalls durch Hydroxyl substituierte Propylengruppe, R⁵ = Wasserstoff und PG gleich C₁-C₅-Acyl oder C₁-C₅-Alkyloxycarbonyl, C₃-C₅-Alkenyloxycarbonyl, besonders bevorzugt R⁴ = Wasserstoff, Methyl, Isopropyl, Isobutyl, Benzyl oder gemeinsam mit R⁵ eine Propylengruppe oder Hydroxypropylengruppe, R⁵ = Wasserstoff und PG gleich Acetyl, Propionyl, Butyryl, Isopropionyl, Isobutyryl oder Methoxycarbonyl, Ethoxycarbonyl, Isopropoxycarbonyl, tert-Butoxycarbonyl oder Allyloxycarbonyl bedeutet und ihre Salze, Solvate oder Solvate der Salze, die α-, β- oder γ-Cyclodextrinclathrate, sowie die mit Liposomen verkapselten Verbindungen.

Besonders bevorzugt sind auch die Verbindungen der Formel la 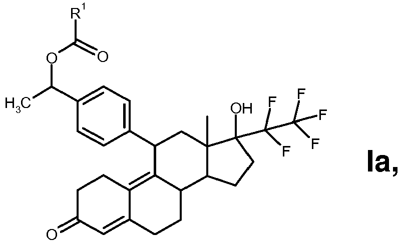 worin
- R¹: C₁-C₁₀-Alkyl, (CH₂)ₙ-Y mit n = 1 - 10 oder CHR⁴NR⁵PG,
- R⁴, R⁵: unabhängig voneinander Wasserstoff, C₁-C₁₀-Alkyl, C₇-C₁₀-Aralkyl, gemeinsam (CH₂)ₘ- mit m = 3 oder 4 oder eine CH₂CHOHCH₂-Gruppe,
- PG: Wasserstoff oder eine Aminoschutzgruppe
- Y: Aryl oder Heteroaryl,
bedeutet und ihre Salze, Solvate oder Solvate der Salze.
Ganz besonders bevorzugt sind die Verbindungen der Formel la in denen R¹ Methyl, Isopropyl, Isobutyl oder Neopentyl bedeutet, insbesondere die Verbindungen: Essigsäure (RS)-1-[4-((8S,11R,13S,14S, 17S)-17-hydroxy-13-methyl-3-oxo-17-pentafluorethyl-2,3,6,7,8,11,12,13,14,15,16, 17-dodecahydro-1*H*-cyclopenta[*a*] phenanthren-11-yl)-phenyl]-ethylester (Bsp. 1)
Isobuttersäure (RS)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*] phenanthren-11-yl)-phenyl]-ethylester (Bsp. 2)
3-Methyl-buttersäure (RS)-1-[4-((8S,11R,13S,14S,17S)-17-hydroxy-13-methyl-3-oxo-17-pentafluorethyl-2,3,6,7,8,11,12,13,14, 15,16,17-dodecahydro-1*H*-cyciopenta[*a*] phenanthren-11-yl)-phenyl]-ethylester (Bsp. 3)
3,3-Dimethyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-ethylester (Bsp. 4)

Ebenfalls ganz besonders bevorzugt sind die Verbindungen der Formel la in denen R¹ (CH₂)₂-Y und Y 2-Methylimidazol-1-yl oder Thiazol-1-yl bedeutet, insbesondere die Verbindungen:
3-(2-Methyl-imidazol-1-yl)-propionsäure (S)-1-[4-((8S,11R, 13S,14S, 17S)-17-hydroxy-13-methyl-3-oxo-17-pentafluorethyl-2,3,6,7,8,11,12,13,14,15,16 17-dodecahydro-1*H-*cyclopenta[*a*]phenanthren-11-yl)-phenyl]-ethylester (Bsp. 14)
3-(2-Methyl-imidazol-1-yl)-propionsäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a] phenanthren-11-yl)-phenyl]-ethylester (Bsp. 14)
3-Thiazol-2-yl-propionsäure (RS)-1-[4-((8S,11R, 13S, 14S, 17S)-17-hydroxy-13-methyl-3-oxo-17-pentafluorethyl-2,3,6,7,8,11,12,13,14,15,16,17-dodecahydro-1*H-*cyclopenta[a]phenanthren-11-yl)-phenyl]-ethylester (Bsp. 15)

Auch ganz besonders bevorzugt sind die Verbindungen der Formel la in denen R¹ CHR⁴NR⁵PG und R⁴ Methyl oder Isopropyl, R⁵ Wasserstoff oder R⁴ gemeinsam mit R⁵ eine Propylengruppe und PG Wasserstoff, tert-Butyloxycarbonyl oder Allyloxycarbonyl bedeutet, insbesondere die Verbindungen:
(S)-2-*tert*-Butoxycarbonylamino-3-methyl-buttersäure (RS)-1-[4-((8S,11R,13S,14S, 17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Bsp. 5)
(R)-2-*tert*-Butoxycarbonylamino-propionsäure (RS)-1-[4-((8S,11R,13S,14S,17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-ethylester (Bsp. 6)
(S)-2-*tert*-Butoxycarbonylamino-propionsäure (RS)-1-[4-((8S,11R,13S,14S,17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-ethylester (Bsp. 7)
(S)-2-Amino-propionsäure (RS)-1-[4-((8S,11R,13S,14S,17S)-17-hydroxy-13-methyl-3-oxo-17-pentafluorethyl-2,3,6,7,8,11,12,13,14, 15,16,17-dodecahydro-1*H-*cyclopenta[a]phenanthren-11-yl)-phenyl]-ethylester (Bsp. 8)
(S)-2-Allyloxycarbonylamino-propionsäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a] phenanthren-11-yl)-phenyl]-ethylester (Bsp. 9)
(S)-2-Allyloxycarbonylamino-propionsäure (S)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a] phenanthren-11-yl)-phenyl]-ethylester (Bsp. 10)
(S)-Pyrrolidine-1,2-dicarbonsäure 1-*tert*-butylester 2-{(RS)-1-[4-((8S,11R,13S,14S, 17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethyl} ester (Bsp. 11)
(S)-Pyrrolidin-2-carbonsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester (Bsp. 12)
(S)-Pyrrolidin-2-carbonsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester Hydrochlorid (Bsp. 13)
(S)-2-Allyloxycarbonylamino-3-methyl-buttersäure (R)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-ethylester (Bsp. 16)

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.
Ganz besonders bevorzugt sind Kombinationen von zwei oder mehreren der oben genannten Vorzugsbereiche.
Ebenfalls besonders bevorzugt sind die erfindungsgemäßen Verbindungen mit einer nach der Vorschrift in Ausführungsbeispiel 19 bestimmten Halbwertszeit in Humanplasma größer 100 Stunden, insbesondere die Verbindungen nach den Beispielen 1, 2, 3, 4, 5, 6, 7, 9, 11, 14A, 15 und 16 (vergl. Beispiel 19, Tabelle 3).

Es wurde gefunden, dass die erfindungsgemäßen Verbindungen bzw. Derivate eine gute, das Progesteron antagonisierende Wirkung aufweisen. In mehreren klinischen Studien wurde gefunden, dass die Behandlung mit Progestronrezeptorantagonisten (Mifepriston, Asoprisnil, Proellex) zu einer signifikanten Schrumpfung von Uterusfibroiden und einer signifikanten Reduktion der mit diesen Uterusfibroiden assoziierten Symptome führen kann. Ferner wurde in klinischen Studien gezeigt, dass unter einer Behandlung mit den genannten Progesteronrezeptorantagonisten auch die von Endometriose verursachten Symptome (insbesondere Schmerzen) deutlich reduziert werden können.
Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung in die einzelnen Verbindungen aufgetrennt werden. Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.
Weiterer Gegenstand der Erfindung ist ein Verfahren zur Herstellung der erfindungsgemäßen Verbindungen, wobei die sekundären Hydroxylgruppen verestert werden (Bsp. 1-7, 9-11 und 14-16). Gegebenenfalls folgt eine Schutzgruppenspaltung (Bsp. 8 und 12).

Die resultierenden Verbindungen der Formel (I) werden gegebenenfalls mit den entsprechenden (i) Lösungsmitteln und/oder (ii) Basen oder Säuren zu ihren Solvaten, Salzen und/oder Solvaten der Salze umsetzt.

Die Erfindung betrifft ferner Verfahren zur Herstellung von Steroidestern der Formel I, dadurch gekennzeichnet, dass man Verbindungen der Formel II, wie in den Beispielen 1 und 5 näher beschrieben verestert und gegebenenfalls in PG vorhandene Schutzgruppen, wie in den Beispielen 8 und 12 näher beschrieben, spaltet. Die Herstellung der erfindungsgemäßen Verbindungen kann durch das folgende Syntheseschema verdeutlicht werden.

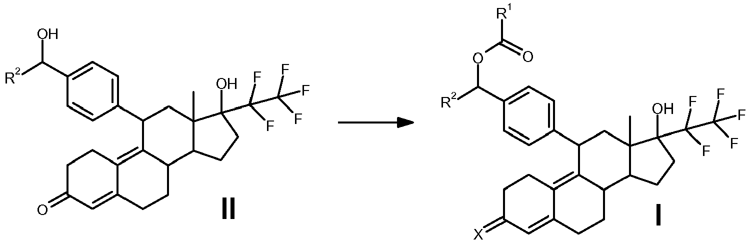

Die Herstellung einer Verbindung der Formel II mit R² = Methyl ist z.B. in WO 98/34947, Beispiel 13 (Seite 22) beschrieben. Die erhaltenen Verbindungen der allgemeinen Formel I in denen X für ein Sauerstoffatom steht, können durch Umsetzung mit Hydroxylaminhydrochlorid Alkyloxyamin-hydrochloriden oder Sulfonylhydrazinen in Gegenwart eines tertiären Amins bei Temperaturen zwischen -20 und +40°C in ihre entprechenden E/Z-konfigurierten Oxime oder Sulfonylhydrazone überführt werden (allgemeine Formel I mit X in der Bedeutung von =NOR³, =NNHSO₂R³). Geeignete tertiäre Basen sind beispielsweise Trimethylamin, Triäthylamin, Pyridin, N,N-Dimethylaminopyridin, 1,5- Diazabicyclo[4.3.0]non-5-en (DBN) und 1,5-Diazabicyclo[5.4.0]undec-5-en (DBU), wobei Pyridin bevorzugt ist. Ein analoges Verfahren ist beispielsweise in WO 98/24801 beschrieben.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Die erfindungsgemäßen Verbindungen zeigen ein nicht vorhersehbares, wertvolles pharmakologisches, pharmakokinetisches und pharmakodynamisches Wirkprofil.
Sie eignen sich daher zur Verwendung als Arzneimittel zur Behandlung und/oder Prophylaxe von Krankheiten bei Menschen und Tieren.
Die pharmazeutische Wirksamkeit der erfindungsgemäßen Verbindungen lässt sich durch ihre Wirkung als Progesteronrezeptorantagonisten, also ihre antagonisierende Wirkung am Progesteronrezeptor erklären.
Weiterer Gegenstand der vorliegenden Erfindung ist der Einsatz der erfindungsgemäßen Verbindungen zur Behandlung und/oder Prophylaxe von Erkrankungen basierend auf hormonabhängigen hyperproliferativen Prozessen, vorzugsweise von gynäkologischen Krankheiten, insbesondere von Uterusfibroiden, der Endometriose oder von hormonabhängigen Mammakarzinomen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.
Weiterer Gegenstand der vorliegenden Erfindung sind die erfindungsgemäßen Verbindungen zur Verwendung in einem Verfahren zur Behandlung und/oder Prophylaxe von Uterusfibroiden, der Endometriose und von hormonabhängigen Mammakarzinomen.
Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.
Weiterer Gegenstand der vorliegenden Erfindung ist ein Verfahren zur Behandlung und/oder Prophylaxe von Erkrankungen, insbesondere der zuvor genannten Erkrankungen, unter Verwendung von 0,1-100 mg der erfindungsgemäßen Verbindungen pro Tag und Patientin bei der Behandlung von Uterusfibroiden oder der Endometriose und für die kontrazeptive Anwendung bzw. von 0,1-500 mg der erfindungsgemäßen Verbindungen pro Tag und Patientin bei Tumor-Erkrankungen (z.B. Menginiome oder hormonabhängige Tumore wie z.B. das Mammakarzinom) und bei der Notfallkontrazeption.
Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.
Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika wie beispielsweise aus der Gruppe der Taxane, Epothilone oder Platinverbindungen.
Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.
In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84tägige Gabe des Progesteronrezeptorantagonisten gefolgt von der 14tägigen Gabe des Gestagens.
Zur Behandlung von mit der Menopause assoziierten Beschwerden kommt eine gleichzeitige oder sequentielle Verabreichung der erfindungsemäßen Verbindungen z.B. mit SERMs, SERDs und Östrogenen in Betracht.
SERMs (Selective Estrogen Receptor Modulators) sind solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen.
Selektive Estrogenrezeptordestabilisatoren (SERD) sind Arzneistoffe, die den Estrogenrezeptor antagonisieren (,reine Antiöstrogene' ohne östrogene Wirkkomponente) und zu einem Abbau des Rezeptors führen (beispielsweise Fulvestrant, ZK-703 und ZK-253 (Hoffmann J et al., J Natl Cancer Inst 2004, 96:210-218) sowie in WO 98/007740, WO 99/33855 und WO 03/045972 beschriebene Verbindungen.
Antiöstrogene sind Verbindungen die den Estrogenrezeptor antagonisieren, beispielsweise Fulvestrant.
Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole.
Kinaseinhibitoren hemmen Enzyme, die einen Phosphatrest von ATP auf andere Substrate, dort insbesondere auf Hydroxygruppen, übertragen, z.B. Sorafenib (Nexavar) oder Imatinib (Gleevec).
Angiogenesehemmer, z.B. Avastin, reduzieren bzw. blockieren die Gefäßneubildung und damit die Durchblutung eines Tumors.
Zytostatika, z.B. cis-Platin, Taxol, Taxotere, Sagopilon, Ixabepilon sind natürliche oder synthetische Substanzen, die Tumorzellen in die Apoptose treiben.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.
Die erfindungsgemäßen Verbindungen können systemisch und/oder lokal wirken. Zu diesem Zweck können sie auf geeignete Weise, wie z.B. oral, intrauterinär, intravaginal, parenteral, pulmonal, nasal, sublingual, lingual, buccal, rectal, dermal, transdermal, conjunctival, otisch oder als Implantat bzw. Stent appliziert werden.
Intrauterinär bedeutet dabei insbesondere die Applikation mittels IUS (intrauterine system) oder IUD (intrauterine device). Die intravaginale Applikation kann u.a. mittels IVR (Vaginalring) erfolgen.
Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nichtsilikon-und/oder Silikonpolymere, insbesondere auch siloxanebasierte Elastomere (vergl. WO 01/47490, insbesondere Seite 7, Zeile 19 - Seite 15, Zeile 15), enthalten.
Für diese Applikationswege können die erfindungsgemäßen Verbindungen in geeigneten Applikationsformen verabreicht werden.
Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.
Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Verschiedene Methoden zur Erhöhung der Löslichkeit schwerlöslicher Wirkstoffe zur Herstellung parenteraler Formulierungen sind in EP 1674098 (Beispiele 1 bis 3) beschrieben. US 6,407,079 beschreibt injizierbare Formulierungen, in denen partiell durch Hydroxyethyl-, Hydroxypropyl-, Dihydroxypropyl-, Methyl- oder Ethylether modifizierte β-Cyclodextrine zur Formulierung von Wirkstoffen eingesetzt werden. Der resultierende Einschluss- oder Adhäsionskomplex ist besser wasserlöslich als der Wirkstoff. Sulfoalkylethercyclodextrine und deren Derivate zur Verbesserung der Löslichkeit wasserunlöslicher Wirkstoffe sind in US 5,376,645 und US 5,134,127 beschrieben.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.
Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien. Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.
Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die folgenden Beispiele dienen der Erläuterung der Erfindung ohne diese in irgend einer Weise zu beschränken.

### Beispiel 1: Essigsäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]-phenanthren-11-yl)-phenyl]-ethylester (A) und (S)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester (B)

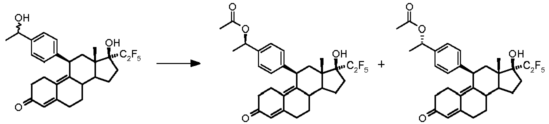

Die Lösung von 1,5 g (2,94 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on (vergl. WO 98/34947, Beispiel 13, Seite 22) in 20 ml Pyridin versetzte man mit 10 ml Essigsäureanhydrid und rührte 2,5 Stunden bei 23°C. Man goss auf gesättigte Natriumhydrogencarbonatlösung, extrahierte mehrfach mit Dichlormethan, wusch die vereinigten organischen Extrakte mit gesättigter Natriumchloridlösung und trocknete über Natriumsulfat. Den nach Filtration und Lösungsmittelabzug erhaltenen Rückstand reinigte man durch Kristallisation aus Diisopropylether. Isoliert wuden 1,47 g (91%) die Titelverbindungen A und B als farbloser kristalliner Feststoff. Eine Trennung der Isomeren erfolgte durch Chromatographie.

¹H-NMR (CDCl₃) von A: δ = 0,59 (3H), 1,27 (3H), 1,39-1,55 (2H), 1,50 (3H), 1,72-1,86 (3H), 2,00-2,10 (2H), 2,22-2,65 (9H), 2,72 (1 H), 4,43 (1 H), 5,78 (1 H), 5,85 (1 H), 7,15 (2H), 7,25 (2H) ppm.

¹H-NMR (CDCl₃) von B: δ = 0,59 (3H), 1,27 (3H), 1,39-1,55 (2H), 1,50 (3H), 1,72-1,86 (3H), 2,0-2,10 (2H), 2,22-2,65 (9H), 2,72 (1 H), 4,43 (1 H), 5,77 (1 H), 5,85 (1 H), 7,14 (2H), 7,25 (2H) ppm.

### Beispiel 2: Isobuttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]-phenanthren-11-yl)-phenyl]-ethylester

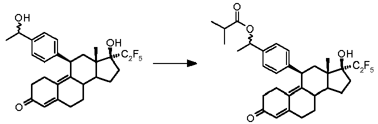

In Analogie zu Beispiel 1 setzte man 150 mg (0,29 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von Isobuttersäureanhydrid um und isolierte nach Aufarbeitung und Reinigung 148 mg (87%) der Titelverbindungen als farblosen Schaum.

¹H-NMR (CDCl₃): δ = 0,58 (3H), 1,10-1,20 (6H), 1,50 (3H), 1,40-1,56 (2H), 1,73-1,87 (3H), 2,00-2,12 (2H), 2,22-2,63 (10H), 2,73 (1 H), 4,44 (1 H), 5,78 (1 H), 5,85 (1 H), 7,14 (2H), 7,25 (2H) ppm.

### Beispiel 3: 3-Methyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

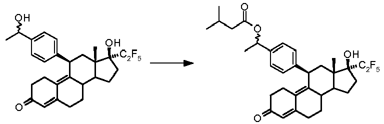

In Analogie zu Beispiel 1 setzte man 150 mg (0,29 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von Isovaleriansäureanhydrid um und isolierte nach Aufarbeitung und Reinigung 157 mg (90%) der Titelverbindungen als farblosen Schaum.

¹H-NMR (CDCl₃): δ = 0,58 (3H), 0,86-0,93 (6H), 1,40-1,56 (2H), 1,51 (3H), 1,73-1,86 (3H), 2,00-2,14 (3H), 2,19 (2H), 2,24-2,63 (9H), 2,73 (1 H), 4,44 (1 H), 5,78 (1 H), 5,87 (1H), 7,14 (2H), 7,26 (2H) ppm.

### Beispiel 4: 3,3-Dimethyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

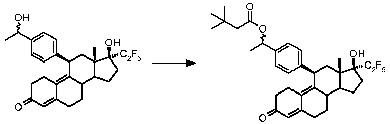

In Analogie zu Beispiel 1 setzte man 150 mg (0,29 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von 3,3-Dimethyl-butyrylchlorid um und isolierte nach Aufarbeitung und Reinigung 113 mg (63%) der Titelverbindungen als farblosen Schaum.

¹H-NMR (CDCl₃): δ = 0,57 (3H), 0,97 (9H), 1,38-1,57 (2H), 1,52 (3H), 1,72-1,87 (3H), 2,00-2,11 (2H), 2,19 (2H), 2,20-2,64 (9H), 2,73 (1 H), 4,43 (1 H), 5,78 (1 H), 5,87 (1 H), 7,14 (2H), 7,28 (2H) ppm.

### Beispiel 5: (S)-2-tert-Butoxycarbonylamino-3-methyl-Buttersäure (RS)-1-[4-((8S,11R, - 13S,14S,17S)-17-hydroxy-13-methyl-3-oxo-17-pentafluorethyl-2,3,6,7,8,11,12,13,14,15, 16,17-dodecahydro-1H-cyclopenta[a]phenanthren-11-yl)-phenyl]-ethylester

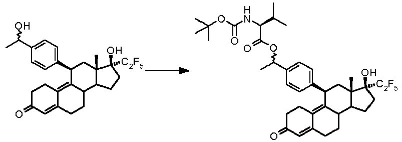

Die Lösung von 300 mg (0,59 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on in 3 ml Pyridin versetzte man mit 14 mg 4-Dimethylaminopyridin, 383 mg (S)-2-tert-Butoxycarbonylamino-3-methyl-butansäure, 130 mg 1-(3-Dimethylaminopropyl)-3-ethylcarbodiimid Hydrochlorid und rührte 16 Stunden bei 23°C. Man goss in Wasser, extrahierte mehrfach mit Dichlormethan, wusch die vereinigten organischen Extrakte mit Wasser und gesättigter Natriumchloridlösung und trocknete über Natriumsulfat. Den nach Filtration und Lösungsmittelabzug erhaltenen Rückstand reinigte man durch Chromatographie. Isoliert wurden 314 mg (75%) der Titelverbindungen als farbloser Schaum.

¹H-NMR (CDCl₃): δ = 0,54+0,59 (3H), 0,63+0,85+0,89+0,95 (6H), 1,43 (9H), 1,53+1,56 (3H), 1,34-1,63 (2H), 1,72-1,87 (3H), 2,00-2,12 (3H) 2,24-2,65 (9H), 2,73 (1H), 4,22 (1 H), 4,44 (1 H), 4,96+5,03 (1 H), 5,78 (1 H), 5,90 (1 H), 7,12-7,19 (2H), 7,24-7,30 (2H) ppm.

### Beispiel 6: (R)-2-tert-Butoxycarbonylamino-propionsäure (RS)-1-[4-((8S,11R,13S, - 14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

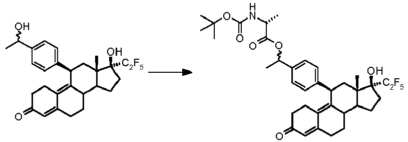

In Analogie zu Beispiel 5 setzte man 300 mg (0,59 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von (R)-2-*tert*-Butoxycarbonylamino-propionsäure um und isolierte nach Aufarbeitung und Reinigung 324 mg (81%) der Titelverbindungen als farblosen Schaum.

¹H-NMR (CDCl₃): δ = 0,57+0,59 (3H), 1,41+1,43 (9H), 1,52+1,54 (3H), 1,29-1,56 (5H), 1,74-1,85 (3H), 2,06 (1 H), 2,12 (1 H), 2,23-2,62 (9H), 2,73 (1 H), 4,31 (1 H), 4,43 (1 H), 5,03 (1 H), 5,78 (1 H), 5,89 (1 H), 7,15 (2H), 7,24+7,25 (2H) ppm.

### Beispiel 7: (S)-2-tert-Butoxycarbonylamino-propionsäure (RS)-1-[4-((8S,11R,13S, - 14S, 17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

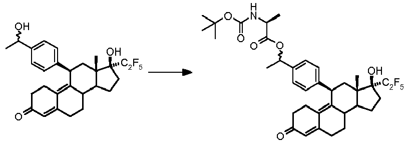

In Analogie zu Beispiel 5 setzte man 300 mg (0,59 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von (S)-2-*tert*-Butoxycarbonylamino-propionsäure um und isolierte nach Aufarbeitung und Reinigung 294 mg (73%) der Titelverbindungen als farblosen Schaum.

¹H-NMR (CDCl₃): δ = 0,57+0,59 (3H), 1,42+1,43 (9H), 1,52+1,54 (3H), 1,29-1,56 (5H), 1,74-1,85 (3H), 2,02-2,13 (2H), 2,22-2,63 (9H), 2,73 (1H), 4,31 (1H), 4,44 (1H), 5,00+5,05 (1H), 5,78 (1H), 5,89 (1H), 7,12-7,18 (2H), 7,22-7,27 (2H) ppm.

### Beispiel 8: (S)-2-Amino-propionsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

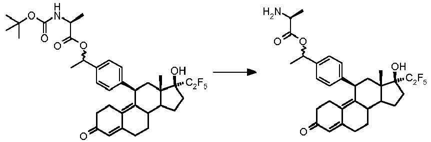

Die Lösung von 50 mg (73 µmol) der nach Beispiel 7 dargestellten Verbindung in 0,5 ml Trifluoressigsäure rührte man 10 Minuten bei 3°C. Man goss auf gesättigte Natriumhydrogencarbonatlösung, extrahierte mehrfach mit Dichlormethan, wusch die vereinigten organischen Extrakte mit gesättigter Natriumchloridlösung und trocknete über Natriumsulfat. Den nach Filtration und Lösungsmittelabzug erhaltenen Rückstand reinigte man durch Chromatographie. Isoliert wurden 26 mg (61 %) der Titelverbindungen als farbloser Schaum.

¹H-NMR (CD₃OD): δ = 0,56 (3H), 1,20-1,58 (10H), 1,67-1,84 (3H), 2,01-2,72 (9H), 2,79 (1 H), 4,51 (1 H), 5,73 (1 H), 5,86 (1 H), 7,16-7,34 (4H) ppm.

### Beispiel 9: (S)-2-Allyloxycarbonylamino-propionsäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester)

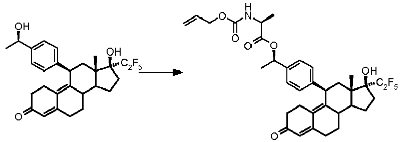

In Analogie zu Beispiel 5 setzte man150 mg (0,29 mmol) (8S,11R,13S,14S,17S)-17-Hydroxy-11-[4-((R)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von (S)-2-Allyloxycarbonylamino-propansäure um und isolierte nach Aufarbeitung und Reinigung 140 mg (72%) der Titelverbindung als farblosen Schaum.

¹H-NMR (CDCl₃): δ = 0,59 (3H), 1,44 (3H), 1,53 (3H), 1,38-1,57 (2H), 1,73-1,87 (3H), 1,99-2,12 (2H), 2,22-2,65 (9H), 2,73 (1H), 4,37 (1H), 4,44 (1H), 4,55 (2H), 5,20 (1H), 5,24-5,35 (2H), 5,78 (1H), 5,82-5,97 (2H), 7,16 (2H), 7,25 (2H) ppm.

### Beispiel 10: (S)-2-Allyloxycarbonylamino-propionsäure (S)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester (B)

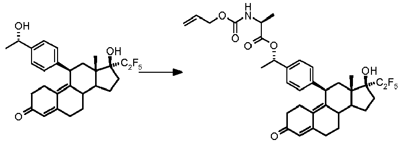

In Analogie zu Beispiel 5 setzte man 150 mg (0,29 mmol) (8S,11R,13S,14S,17S)-17-Hydroxy-11-[4-((S)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von (S)-2-Allyloxycarbonylamino-propansäure um und isolierte nach Aufarbeitung und Reinigung 87 mg (44%) der Titelverbindung als farblosen Schaum.

¹H-NMR (CDCl₃): δ = 0,57 (3H), 1,34 (3H), 1,47 (2H), 1,55 (3H), 1,75-1,86 (3H), 2,02-2,11 (2H), 2,23-2,63 (9H), 2,73 (1 H), 4,39 (1 H), 4,44 (1 H), 4,56 (2H), 5,17-5,35 (3H), 5,78 (1H), 5,85-5,96 (2H), 7,15 (2H), 7,24 (2H) ppm.

### Beispiel 11: (S)-Pyrrolidine-1,2-dicarbonsäure 1-tert-butylester 2-{(RS)-1-[4-((8S,11R, - 13S, 14S, 17S)-17-hydroxy-13-methyl-3-oxo-17-pentafluorethyl-2,3,6,7,8,11,12,13,14,15, 16,17-dodecahydro-1H-cyclopenta[a]phenanthren-11-yl)-phenyl]-ethyl} ester

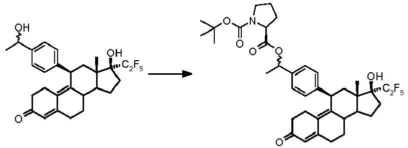

In Analogie zu Beispiel 5 setzte man 330 mg (0,65 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von (S)-Pyrrolidine-1,2-dicarbonsäure-1-tert-butylester um und isolierte nach Aufarbeitung und Reinigung 356 mg (78%) der Titelverbindungen als farblosen Schaum.

¹H-NMR (CDCl₃): δ = 0,57 (3H), 1,34-1,56 (14H), 1,74-1,97 (6H), 2,02-2,63 (12H), 2,73 (1 H), 3,36-3,57 (2H), 4,24+4,34 (1 H), 4,43 (1 H), 4,78 (1 H), 5,90 (1 H), 7,16 (2H), 7,26 (2H) ppm.

### Beispiel 12: (S)-Pyrrolidine-2-carbonsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

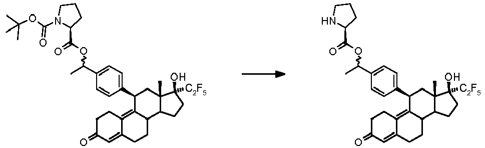

110 mg (0,16 mmol) der nach Beispiel 11 dargestellten Verbindung versetzt man mit 0,44 ml einer 4 molaren Lösung von Chlorwasserstoff in Dioxan und rührte 20 Minuten bei 23°C. Man goss auf gesättigte Natriumhydrogencarbonatlösung, extrahierte mehrfach mit Dichlormethan, wusch die vereinigten organischen Extrakte mit gesättigter Natriumchloridlösung und trocknete über Natriumsulfat. Den nach Filtration und Lösungsmittelabzug erhaltenen Rückstand reinigte man durch Chromatographie. Isoliert wurden 29 mg (31%) der Titelverbindungen als farbloser Schaum.

¹H-NMR (CDCl₃): δ = 0,56+0,57 (3H), 1,40-2,64 (21 H), 1,52 (3H), 2,73 (1 H), 2,87 (1 H), 3,05 (1 H), 3,75 (1 H), 4,44 (1 H), 5,78 (1 H), 5,89 (1 H), 7,15 (2H), 7,25 (2H) ppm.

### Beispiel 13: (S)-Pyrrolidine-2-carbonsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester Hydrochlorid

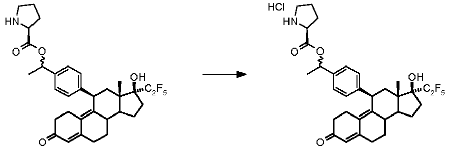

Die Lösung von 29 mg (48 µmol) der nach Beispiel 12 dargestellten Verbindung in 0,5 ml Dichlormethan versetzte man mit 13 µl einer 4 molaren Lösung von Chlorwasserstoff in Dioxan und engt zur Trockne ein. Isoliert wurden 30 mg (98%) der Titelverbindungen als farbloser Schaum.

¹H-NMR (CDCl₃): δ = 0,55+0,58 (3H), 1,38-1,66 (5H), 1,57 (3H), 1,71-1,88 (3H), 1,90-2,66 (13H), 2,73 (1 H), 3,44 (2H), 4,31-4,49 (2H), 5,78 (1 H), 5,93 (1 H), 7,17 (2H), 7,25 (2H) ppm.

### Beispiel 14: 3-(2-Methyl-imidazol-1-yl)-propionsäure (S)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester (A) und 3-(2-Methyl-imidazol-1-yl)-propionsäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester (B)

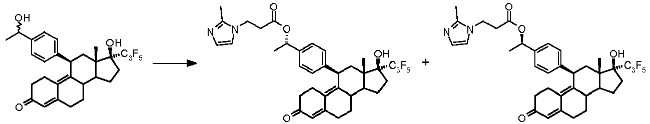

In Analogie zu Beispiel 5 setzte man 150 mg (0,29 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von 3-(2-Methyl-1*H*-imidazol)-propionsäure um und isolierte nach Aufarbeitung und chromatographischer Trennung 67 mg (35%) der Titelverbindung A sowie 52 mg (27%) der Titelverbindung B, jeweils als farblosen Schaum.

¹H-NMR (CDCl₃) von A: δ = 0,51 (3H), 1,43-1,57 (2H), 1,53 (3H), 1,69-1,94 (4H), 2,08 (1 H), 2,25 (3H), 2,27-2,60 (7H), 2,62-2,76 (3H), 2,82 (1 H), 2,97 (1 H), 3,90 (1 H), 4,29 (1 H), 4,39 (1 H), 5,64 (1 H), 5,79 (1 H), 6,87 (1 H), 6,92 (1 H), 6,96 (2H), 7,00 (2H) ppm.

¹H-NMR (CDCl₃) von B: δ = 0,55 (3H), 1,44-1,58 (2H), 1,57 (3H), 1,74-1,92 (4H), 2,09 (1 H), 2,29 (3H), 2,25-2,81 (12H), 4,09 (1 H), 4,24 (1 H), 4,45 (1 H), 5,81 (1 H), 6,00 (1 H), 5,85 (2H), 7,11 (2H), 7,20 (2H) ppm.

### Beispiel 15: 3-Thiazol-2-yl-propionsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester

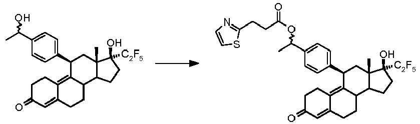

In Analogie zu Beispiel 5 setzte man 150 mg (0,29 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15,16,17-dodecahydro-cyclopenta[a]phenanthren-3-on unter Verwendung von 3-(2-Thiazolyl)-propionsäure um und isolierte nach Aufarbeitung und Reinigung 119 mg (62%) der Titelverbindungen als farblosen Schaum.

¹H-NMR (CDCl₃): δ = 0,59 (3H), 1,40-1,55 (2H), 1,49 (3H), 174-1,86 (3H), 2,06 (1H), 2,18 (1 H), 2,23-2,63 (9H), 2,73 (1 H), 2,81-2,93 (2H), 3,33 (2H), 4,43 (1 H), 5,78 (1 H), 5,88 (1 H), 7,13 (2H), 7,19 (1 H), 7,23 (2H), 7,66 (1 H) ppm.

### Beispiel 16: (S)-2-Allyloxycarbonylamino-3-methyl-buttersäure (RS)-1-[4-((8S,11R,13S, 14S, 17S)-17-hydroxy-13-methyl-3-oxo-17-pentafluorethyl-2,3,6,7,8,11,12,13,14,15,16,17-dodecahydro-1H-cyclopenta[a] phenanthren-11-yl)-phenyl]-ethylester

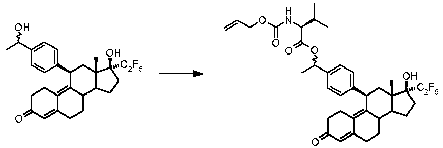

In Analogie zu Beispiel 5 setzte man 400 mg (0,78 mmol) (8S,11R,13S,14S,17S)-17-Hydroxy-11-[4-((RS)-1-hydroxy-ethyl)-phenyl]-13-methyl-17-pentafluorethyl-1,2,6,7,8,11,12,13,14,15, 16,17-dodecahydro-cyclopenta[a]phenanthren-3-on unter Verwendung von (S)-2-Allyloxycarbonylamino-3-methyl-buttersäure um und isolierte nach Aufarbeitung und Reinigung 495 mg (91%) der Titelverbindungen als farblosen Schaum.

¹H-NMR (CDCl₃): δ = 0,55+0,60 (3H), 0,74-1,26 (7H), 1,37-1,63 (5H), 1,73-1,88 (3H), 2,03-2,66 (11 H), 2,74 (1 H), 4,30 (1 H), 4,40 (1 H), 4,57 (2H), 5,16-5,38 (3H), 5,80 (1 H), 5,84-6,01 (2H), 7,16 (2H), 7,27 (2H) ppm.

### Beispiel 17: Bestimmung der hydrolytischen Stabilität

Die zu untersuchenden Ester löste man jeweils in einem 5:1-Gemisch aus Dioxan und Pufferlösung (pH 1,2, pH 5,0 oder pH 8,0) bei 23°C. Nach unterschiedlichen Zeiten wurde der Gehalt an Ester und Hydrolyseprodukten (8S,11R,13S,14S,17S)-17-Hydroxy-11-[4-((R)-1-hydroxy-ethyl)-phenyl]-13-methyl-17-pentafluorethyl-1,2,6,7,8,11,12,13,14,15,16, 17-dodecahydro-cyclopenta[a]phenanthren-3-on und/oder (8S,11R,13S,14S,17S)-17-Hydroxy-11-[4-((S)-1-hydroxy-ethyl)-phenyl]-13-methyl-17-pentafluorethyl-1,2,6,7,8,11,12, 13,14,15,16,17-dodecahydro-cyclopenta[a]phenanthren-3-on per HPLC bestimmt. Der Versuch wurde nach 24 Stunden oder 192 Stunden beendet.

**Tabelle 1: Hydrolytische Stabilität bei 23°C und unterschiedlichem pH-Wert**

| Beispiel | t_{1/2} [h] @ 23°C | | |
|---|---|---|---|
| | pH = 1,2 | pH = 5,0 | pH = 8,0 |
| 1 | >>24* | >>24* | >>24* |
| 2 | >>24* | >>24* | >>24* |
| 3 | >>24* | >>24* | >>24* |
| 4 | >>24* | >>24* | >>24* |
| 5 | >>192* | >>192* | >>192* |
| 6 | >>24* | >>24* | >>24* |
| 7 | >>24* | >>24* | >>24* |
| 9 + 10 | >>192* | >>192* | >>192* |
| 11 | >>192* | nb** | >>192* |
| 13 | >>192* | nb | >>192* |
| 14 | >>192* | nb | >>192* |
| 15 | >>192* | nb | >>192* |
| 16 | >>192* | nb | >>192* |

| | | | |
|---|---|---|---|
| *Nach 24 Stunden bzw. 192 Stunden ist der Gehalt an Hydrolyseprodukt noch < 1 %. **nb: nicht bestimmt | | | |

### Beispiel 18: Bestimmung der hydrolytischen Stabilität in künstlichem Magensaft

Die zu untersuchenden Ester löste man jeweils in einer Pufferlösung von pH 1,2, die mit Pepsin versetzt ist, bei 37°C. Nach unterschiedlichen Zeiten wurde der Gehalt an Ester und Hydrolyseprodukten (8S,11R,13S,14S,17S)-17-Hydroxy-11-[4-((R)-1-hydroxyethyl)-phenyl]-13-methyl-17-pentafluorethyl-1,2,6,7,8,11,12,13,14,15,16,17-dodecahydro-cyclopenta[a]phenanthren-3-on und/oder (8S, 11 R,13S,14S,17S)-17-Hydroxy-11-[4-((S)-1-hydroxy-ethyl)-phenyl]-13-methyl-17-pentafluorethyl-1,2,6,7,8,11,12,13,14,15,16,17-dodecahydro-cyclopenta[a]phenanthren-3-on per HPLC bestimmt. Der Versuch wurde nach 24 Stunden beendet.

**Tabelle 2: Hydrolytische Stabilität bei 37°C in künstlichem Magensaft (pH 1,2)**

| Beispiel | t_{1/2} [h] @ 37°C |
|---|---|
| 1 | 9,6 |
| 2 | >>24* |
| 3 | >>24* |
| 4 | >>24* |
| 5 | 3,0 |
| 6 | 5,4 |
| 7 | 4,4 |

| | |
|---|---|
| *Nach 24 Stunden ist der Gehalt an Hydrolyseprodukt noch < 1 %. | |

### Beispiel 19: Bestimmung der Stabilität in humanem und Ratten-Plasma

Es wurde eine Stammlösung von 1,0 mg der zu testenden Substanz in einem 9:1-Gemisch aus Acetonitril und Dimethylsulfoxid hergestellt. 20 µl dieser Stammlösung wurden bei 37°C zu 1 ml Human- oder Rattenplasma gegeben. Für die Gehaltsbestimmung wurden jeweils 100 µl Proben zu unterschiedlichen Zeitpunkten gezogen. Die Enzymaktivität wurde durch Zugabe von 300 µl Acetonitril blockiert, die Proben wurden über 10 Minuten bei 5000 U/min zentrifugiert und im Überstand der Gehalt an Ester und Hydrolyseprodukten (8S,11R,13S, 14S,17S)-17-Hydroxy-11-[4-((R)-1-hydroxy-ethyl)-phenyl]-13-methyl-17-pentafluorethyl-1,2,6,7,8,11,12,13,14,15,16,17-dodecahydro-cyclopenta[a]phenanthren-3-on und/oder (8S,11R,13S,14S,17S)-17-Hydroxy-11-[4-((S)-1-hydroxy-ethyl)-phenyl]-13-methyl-17-pentafluorethyl-1,2,6,7,8,11,12, 13,14,15,16,17-dodecahydro-cyclopenta[a]phenanthren-3-on per HPLC bestimmt. Aus den Konzentrations-Zeit-Kurven wurde die Abbaukinetik berechnet. Die Stabilität der getesteten Substanz wurde als Halbwertzeit angegeben.

**Tabelle 3: Stabilität bei 37°C in humanem und Ratten-Plasma**

| Beispiel | t_{1/2} [h] @ 37°C | |
|---|---|---|
| | Human | Ratte |
| 1 | 609 | 57,5 |
| 2 | 803 | 33,4 |
| 3 | >1000 | 89 |
| 4 | >1000 | >1000 |
| 5 | >1000 | 156 |
| 6 | 491 | 43,9 |
| 7 | >1000 | 3,1 |
| 9 | 231 | 0,3 |
| 10 | 22,9 | 3,6 |
| 11 | >1000 | 462 |
| 13 | 9,7 | 5,3 |
| 14A | 190 | 48,5 |
| 14B | 19,8 | 0,8 |
| 15 | 131 | 1,2 |
| 16 | >1000 | 33,2 |

### Beispiel 20: Progesteronerezeptor-antagonistische Wirkung in stabilen Transfektanten humaner Neuroblastoma-Zellen (SK-N-MC Zellen) mit dem humanem Progesteron A-oder Progesteron B-Rezeptor und einem MN-LUC Reporter Konstrukt

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0,01 nmol/l, 0,1 nmol/l, 1 nmol/l, 10 nmol/l, 100 nmol/l und 1 pmol/l) inkubiert, um die agonistische Wirksamkeit zu bestimmen. Als Positivkontrolle der Reportergeninduktion wurden die Zellen mit dem synthetischen Gestagen Promegeston (0,01 nmol/l, 0,1 nmol/l, 1 nmol/l, 10 nmol/l, 100 nmol/l and 1 pmol/l) behandelt. Zur Bestimmung der antagonistischen Aktivitat wurden die Zellen mit 0,1 nmol/l Promegeston und zusätzlich mit steigenden Mengen der jeweiligen Testverbindung (0,01 nmol/l, 0,1 nmol/l, 1 nmol/l, 10 nmol/l, 100 nmol/l and 1 pmol/l) behandelt. Die Aktivität des Reportergens LUC (LUC = Luciferase) wurde in den Zellysaten bestimmt und als RLU (relative light units) gemessen. Alle Meßwerte werden angegeben als % Wirksamkeit und als EC₅₀ bzw. IC₅₀ Konzentrationen.
a) agonistische Aktivität:
   Keine der genannten Verbindungen zeigt eine agonistische Aktivität.
b) antagonistische Aktivität:
   Alle genannten Verbindungen zeigen eine 100%ige antagonistische Wirksamkeit. Die antagonistische Wirkstärke der Verbindungen ist in Tabelle 4 zusammengefasst.

**Tabelle 4: Antagonistische Wirkstärke der Verbindungen**

| Bsp. | PR-A IC₅₀ [nM] | PR-B IC₅₀ [nM] | Bsp. | PR-A IC₅₀ [nM] | PR-B IC₅₀ [nM] |
|---|---|---|---|---|---|
| 1 | 0,07 | 0,08 | 10 | 0,08 | 0,09 |
| 2 | 0,1 | 0,1 | 11 | 0,1 | 0,05 |
| 3 | 0,21 | 0,20 | 12 | nb | nb |
| 4 | 0,2 | 0,4 | 13 | 0,27 | 0,74 |
| 5 | 0,3 | 0,8 | 14A | 0,18 | 0,29 |
| 6 | 0,1 | 0,2 | 14B | 0,29 | 0,15 |
| 7 | 0,14 | 0,10 | 15 | 0,1 | 0,1 |
| 8 | nb | nb | 16 | 0,11 | 0,71 |
| 9 | 0,01 | 0,09 | nb: nicht bestimmt | | |

## Patentansprüche

1. Verbindung der Formel I 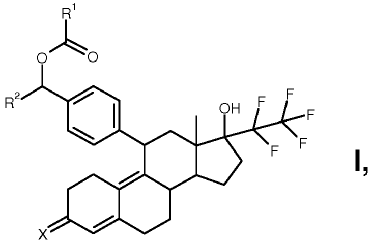 in welcher
X Sauerstoff, eine Gruppe NOR³ oder =NNHSO₂R³,
R¹ C₁-C₁₀-Alkyl, (CH₂)ₙ-Y oder CHR⁴NR⁵PG,
R² Wasserstoff oder C₁-C₁₀-Alkyl,
R³ Wasserstoff, C₁-C₁₀-Alkyl, Aryl oder C₇-C₂₀-Aralkyl,
n 1 bis 10,
Y Wasserstoff, Aryl oder Heteroaryl,
R⁴, R⁵ unabhängig voneinander Wasserstoff, C₁-C₁₀-Alkyl, C₇-C₁₀-Aralkyl, gemeinsam eine (CH₂)ₘ-oder eine CH₂CHOHCH₂-Gruppe,
m 3 oder 4,
PG Wasserstoff oder eine Aminoschutzgruppe
bedeutet und ihre Salze, Solvate oder Solvate der Salze, einschließlich aller Kristallmodifikationen, die α-, β- oder γ-Cyclodextrinclathrate, sowie die mit Liposomen verkapselten Verbindungen
worin
Aryl steht für einen mono- bis tricyclischen aromatischen, substituierten oder unsubstituierten carbocyclischen Rest, die einfach oder mehrfach mit Halogen (F, Cl, Br, I), OH, O-Alkyl, CO₂H, CO₂-Alkyl, NH₂, NH(C₁-C₁₀-Alkyl), N(C₁-C₁₀-Alkyl)₂, insbesondere N(CH₃)₂, NO₂, N₃, CN, C₁-C₁₀-Alkyl, C₂-C₁₀-Alkylen, C₂-C₁₀-Alkinyl, C₁-C₁₀-Perfluor-Alkyl, C₁-C₁₀-Acyl, C₁-C₁₀-Acyloxy-Gruppen, substituiert sein können,
Heteroaryl steht für einen aromatischen, mono- oder bicyclischen Rest mit in der Regel 5 bis 10, und bis zu 5 Heteroatomen aus der Reihe S, O und N, beispielhaft, die einfach mit C₁-C₄-Alkyl substituiert sein können,
Aralkyl steht für Aralkylgruppen und können einfach oder mehrfach durch Halogen, OH, O-Alkyl, CO₂H, CO₂-Alkyl, NH₂, NH(C₁-C₁₀-Alkyl), N(C₁-C₁₀-Alkyl)₂, NO₂, N₃, CN, C₁-C₂₀-Alkyl, C₁-C₁₀-Perfluor-Alkyl, C₁-C₂₀-Acyl, C₁-C₂₀-Acyloxy-Gruppen substituiert sein.

2. Verbindungen nach Anspruch 1 worin R¹ Methyl, Isopropyl, Isobutyl oder Neopentyl bedeutet.

3. Verbindungen nach Anspruch 1 worin R¹ (CH₂)ₙ-Y mit n = 1 - 10 bedeutet und Y ein aromatischer, mono- oder bicyclischer Rest mit 5 bis 10 Ringatomen und mit bis zu 5 Heteroatomen aus der Reihe S, O und N ist.

4. Verbindungen nach Anspruch 3, worin n = 1 - 5 bedeutet und Y ein aromatischer, mono- oder bicyclischer Rest mit 5 bis 9 Ringatomen und mit bis zu 4 Heteroatomen aus der Reihe S, O und N ist.

5. Verbindungen nach Anspruch 4, worin n = 1 - 3 bedeutet und Y = Imidazolyl, Thiazolyl oder Pyridyl ist.

6. Verbindungen nach Anspruch 1, worin R¹ ein Aminosäurerest CHR⁴NR⁵PG ist und R⁴ Wasserstoff, C₁-C₅-Alkyl, C₇-C₁₀-Aralkyl, oder gemeinsam mit R⁵ eine, gegebenenfalls durch Hydroxyl substituierte Ethylen- oder Propylengruppe, R⁵ Wasserstoff und PG C₁-C₅-Acyl, C₁-C₅-Alkyloxycarbonyl oder C₃-C₅-alkenyloxycarbonyl bedeutet.

7. Verbindungen nach Anspruch 6, worin R⁴ Wasserstoff, C₁-C₄-Alkyl, C₇-C₈-Aralkyl, oder gemeinsam mit R⁵ eine, gegebenenfalls durch Hydroxyl substituierte Ethylengruppe, R⁵ = Wasserstoff und PG C₁-C₅-Acyl, C₁-C₅-Alkyloxycarbonyl oder C₃-C₅- alkenyloxycarbonyl bedeutet.

8. Verbindungen nach Anspruch 7, worin R⁴ Wasserstoff, Methyl, Isopropyl, Isobutyl, Benzyl oder gemeinsam mit R⁵ eine Ethylengruppe oder Hydroxyethylengruppe, R⁵ = Wasserstoff und PG gleich Acetyl, Propionyl, Butyryl, Isopropionyl, Isobutyryl, Methoxycarbonyl, Ethoxycarbonyl, Isopropoxycarbonyl, tert.-Butoxycarbonyl oder Allyloxycarbonyl bedeutet.

9. Verbindungen nach Anspruch 1 der Formel 1a 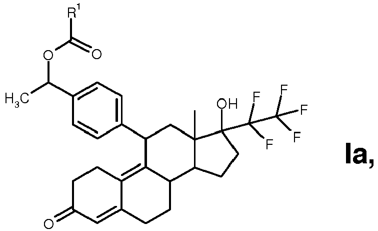 worin
R¹ C₁-C₁₀-Alkyl, (CH₂)ₙ-Y mit n = 1 - 10 oder CHR⁴NR⁵PG,
R⁴, R⁵ unabhängig voneinander Wasserstoff, C₁-C₁₀-Alkyl, C₇-C₁₀-Aralkyl, gemeinsam (CH₂)ₘ- mit m = 3 oder 4 oder eine CH₂CHOHCH₂-Gruppe,
PG Wasserstoff oder eine Aminoschutzgruppe
Y Aryl oder Heteroaryl,
bedeutet und ihre Salze, Solvate oder Solvate der Salze.

10. Verbindungen nach Anspruch 9 worin R¹ Methyl, Isopropyl, Isobutyl oder ^{tert.}Butyl bedeutet.

11. Verbindungen nach Anspruch 9 worin R¹ (CH₂)₂-Y und Y 2-Methylimidazol-1-yl oder Thiazol-1-yl bedeutet.

12. Verbindungen nach Anspruch 9 worin R¹ CHR⁴NR⁵PG und R⁴ Methyl oder Isopropyl, R⁵ Wasserstoff oder R⁴ gemeinsam mit R⁵ eine Gruppe -(CH₂)₃- und PG Wasserstoff, -COOC(CH₃)₃ oder -COOCH₂CH=CH₂ bedeutet.

13. Die Verbindungen nach einem der vorstehenden Ansprüche, nämlich
Essigsäure (RS)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*] phenanthren-11-yl)-phenyl]-ethylester (Beispiel 1)
Isobuttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a] phenanthren-11-yl)-phenyl]-ethylester (Beispiel 2)
3-Methyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a] phenanthren-11-yl)-phenyl]-ethylester (Beispiel 3)
3,3-Dimethyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 4)
3-(2-Methyl-imidazol-1-yl)-propionsäure (S)-1-[4-((8S,11R,13S,14S, 17S)-17-hydroxy-13-methyl-3-oxo-17-pentafluorethyl-2,3,6,7,8,11, 12,13,14,15,16 17-dodecahydro-1*H*-cyclopenta[*a*]phenanthren-11-yl)-phenyl]-ethylester (Beispiel 14)
3-(2-Methyl-imidazol-1-yl)-propionsäure (R)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-ethylester (Beispiel 14)
3-Thiazol-2-yl-propionsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 15)
(S)-2-*tert*-Butoxycarbonylamino-3-methyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 5)
(R)-2-*tert*-Butoxycarbonylamino-propionsäure (RS)-1-[4-((8S,11R,13S,14S, 17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-ethylester (Beispiel 6)
(S)-2-*tert*-Butoxycarbonylamino-propionsäure (RS)-1-[4-((8S,11R,13S,14S, 17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-ethylester (Beispiel 7)
(S)-2-Amino-propionsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 8)
(S)-2-Allyloxycarbonylamino-propionsäure (R)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-ethylester (Beispiel 9)
(S)-2-Allyloxycarbonylamino-propionsäure (S)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-ethylester (Beispiel 10)
(S)-Pyrrolidine-1,2-dicarbonsäure 1-*tert*-butylester 2-{(RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethyl} ester (Beispiel 11)
(S)-Pyrrolidin-2-carbonsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester (Beispiel 12)
(S)-Pyrrolidin-2-carbonsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1H-cyclopenta[a]phenanthren-11-yl)-phenyl]-ethylester Hydrochlorid (Beispiel 13)
(S)-2-Allyloxycarbonylamino-3-methyl-buttersäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 16).

14. Verbindungen nach Anspruch1 nämlich
Essigsäure (RS)-1-[4-((8S,11R,13S,14S, 17S)-17-hydroxy-13-methyl-3-oxo-17-pentafluorethyl-2,3,6,7,8,11,12,13,14,15,16,17-dodecahydro-1*H*-cyclopenta[*a*] phenanthren-11-yl)-phenyl]-ethylester (Beispiel 1),
Isobuttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[a] phenanthren-11-yl)-phenyl]-ethylester (Beispiel 2),
3-Methyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H-*cyclopenta[*a*]phenanthren-11-yl)-phenyl]-ethylester (Beispiel 3),
3,3-Dimethyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 4),
(S)-2-*tert*-Butoxycarbonylamino-3-methyl-buttersäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 5),
(R)-2-*tert*-Butoxycarbonylamino-propionsäure (RS)-1-[4-((8S,11R,13S,14S, 17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-ethylester (Beispiel 6),
(S)-2-*tert*-Butoxycarbonylamino-propionsäure (RS)-1-[4-((8S,11R,13S,14S, 17S)-17-hydroxy-13-methyl-3-oxo-17-pentafluorethyl-2,3,6,7,8,11,12,13,14,15, 16,17-dodecahydro-1*H*-cyclopenta[*a*]phenanthren-11-yl)-phenyl]-ethylester (Beispiel 7),
(S)-2-Allyloxycarbonylamino-propionsäure (R)-1-[4-((8S,11R,13S,14S,17S)-17-hydroxy-13-methyl-3-oxo-17-pentafluorethyl-2,3,6,7,8,11,12,13,14,15,16, 17-dodecahydro-1*H*-cyclopenta[*a*]phenanthren-11-yl)-phenyl]-ethylester (Beispiel 9),
(S)-Pyrrolidine-1,2-dicarbonsäure 1-*tert*-butylester 2-{(RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethyl} ester (Beispiel 11),
3-(2-Methyl-imidazol-1-yl)-propionsäure (S)-1-[4-((8S,11R,13S,14S,17S)-17-hydroxy-13-methyl-3-oxo-17-pentafluorethyl-2,3,6,7,8,11, 12,13,14,15,16 17-dodecahydro-1*H*-cyclopenta[*a*]phenanthren-11-yl)-phenyl]-ethylester (Beispiel. 14A),
3-Thiazol-2-yl-propionsäure (RS)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel. 15),
(S)-2-Allyloxycarbonylamino-3-methyl-buttersäure (R)-1-[4-((8S,11R,13S,14S,17S)-17-hydroxy-13-methyl-3-oxo-17-pentafluorethyl-2,3,6,7,8,11,12,13,14,15,16,17-dodecahydro-1*H*-cyclopenta[*a*]phenanthren-11-yl)-phenyl]-ethylester (Beispiel 16)
mit einer in vitro ermittelten Halbwertszeit in Humanplasma größer 100 Stunden.

15. Verbindung, wie in einem der Ansprüche 1 bis 14 definiert, zur Behandlung und/oder Prophylaxe von Krankheiten.

16. Verbindung, wie in einem der Ansprüche 1 bis 14 definiert, zur Behandlung und/oder Prophylaxe von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden.

17. Verbindung, wie in einem der Ansprüche 1 bis 14 definiert, zur Fertilitätskontrolle und Notfallkontrazeption.

18. Verwendung einer Verbindung, wie in einem der Ansprüche 1 bis 14 definiert, zur Herstellung eines Arzneimittels zur Behandlung und/oder Prophylaxe von Krankheiten.

19. Verwendung einer Verbindung, wie in einem der Ansprüche 1 bis 14 definiert, zur Herstellung eines Arzneimittels zur Behandlung und/oder Prophylaxe von Uterusfibroiden (Myomen, uterine Leiomyome), der Endometriose, schweren Menstruationsblutungen, Meningiomen, hormonabhängigen Mammakarzinomen und von mit der Menopause assoziierten Beschwerden.

20. Verwendung einer Verbindung, wie in einem der Ansprüche 1 bis 14 definiert, zur Herstellung eines Mittels zur Fertilitätskontrolle und Notfallkontrazeption.

21. Arzneimittel enthaltend eine Verbindung, wie in einem der Ansprüche 1 bis 14 definiert, in Kombination mit einem oder mehreren inerten, nichttoxischen, pharmazeutisch geeigneten Hilfsstoff.

22. Arzneimittel enthaltend eine Verbindung, wie in einem der Ansprüche 1 bis 14 definiert, in Kombination mit einem oder mehreren weiteren Wirkstoffen.

23. Arzneimittel nach Anspruch 22, wobei der weitere Wirkstoff ausgewählt ist aus der Gruppe der SERMs, SERDs, Antiöstrogene, Aromataseinhibitoren, Kinaseinhibitoren, Angiogeneseinhibitoren und/oder Cytostatika.

24. Arzneimittel nach Anspruch 22, wobei der weitere Wirkstoff ausgewählt ist aus der Gruppe der Gestagene oder Gestagen/Östrogen-Kombinationen.

25. Arzneimittel nach einem der Ansprüche 21 bis 24, zur Behandlung und Prophylaxe von Uterusfibroiden (Myomen, uterine Leiomyome), der Endometriose, schwerer Menstruationsblutungen, Meningiomen, hormonabhängigen Mammakarzinomen und von mit der Menopause assoziierten Beschwerden.

## Claims

1. Compound of the formula I 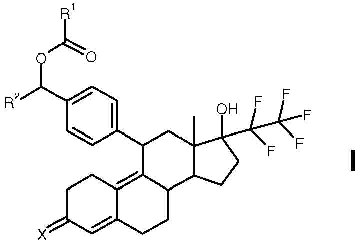 in which
X is oxygen or an NOR³ or =NNHSO₂R³ group,
R¹ is C₁-C₁₀-alkyl, (CH₂)ₙ-Y or CHR⁴NR⁵PG,
R² is hydrogen or C₁-C₁₀-alkyl,
R³ is hydrogen, C₁-C₁₀-alkyl, aryl or C₇-C₂₀-aralkyl,
n is 1 to 10,
Y is hydrogen, aryl or heteroaryl,
R⁴, R⁵ are each independently hydrogen, C₁-C₁₀-alkyl, C₇-C₁₀-aralkyl, or together are a (CH₂)ₘ- or a CH₂CHOHCH₂- group,
m is 3 or 4,
PG is hydrogen or an amino protecting group,
and the salts, solvates or solvates of the salts thereof, including all crystal polymorphs, the α-, β- or γ-cyclodextrin clathrates, and the compounds encapsulated with liposomes,
in which
aryl is a mono- to tricyclic aromatic substituted or unsubstituted carbocyclic radical, which may be mono- or polysubstituted by halogen (F, Cl, Br, I), OH, O-alkyl, CO₂H, CO₂-alkyl, NH₂, NH(C₁-C₁₀-alkyl), N(C₁-C₁₀-alkyl)₂, especially N(CH₃)₂, NO₂, N₃, CN, C₁-C₁₀-alkyl, C₂-C₁₀-alkylene, C₂-C₁₀-alkynyl, C₁-C₁₀-perfluoroalkyl, C₁-C₁₀-acyl, C₁-C₁₀-acyloxy groups,
heteroaryl is an aromatic mono- or bicyclic radical having generally 5 to 10 and up to 5 heteroatoms from the group of S, O and N, by way of example, which may be monosubstituted by C₁-C₄-alkyl,
aralkyl represents aralkyl groups and may be mono-or polysubstituted by halogen, OH, O-alkyl, CO₂H, CO₂-alkyl, NH₂, NH (C₁-C₁₀-alkyl), N(C₁-C₁₀-alkyl)₂, NO₂, N₃, CN, C₁-C₂₀-alkyl, C₁-C₁₀-perfluoroalkyl, C₁-C₂₀-acyl, C₁-C₂₀-acyloxy groups.

2. Compounds according to Claim 1, in which R¹ is methyl, isopropyl, isobutyl or neopentyl.

3. Compounds according to Claim 1, in which R¹ is (CH₂)ₙ-Y where n = 1-10 and Y is an aromatic mono-or bicyclic radical having 5 to 10 ring atoms and having up to 5 heteroatoms from the group of S, O and N.

4. Compounds according to Claim 3, in which n = 1-5 and Y is an aromatic mono- or bicyclic radical having 5 to 9 ring atoms and having up to 4 heteroatoms from the group of S, O and N.

5. Compounds according to Claim 4, in which n = 1-3 and Y = imidazolyl, thiazolyl or pyridyl.

6. Compounds according to Claim 1, in which R¹ is an amino acid radical CHR⁴NR⁵PG and R⁴ is hydrogen, C₁-C₅-alkyl, C₇-C₁₀-aralkyl, or together with R⁵ is an optionally hydroxyl-substituted ethylene or propylene group, R⁵ is hydrogen and PG is C₁-C₅-acyl, C₁-C₅-alkyloxycarbonyl or C₃-C₅-alkenyloxycarbonyl.

7. Compounds according to Claim 6, in which R⁴ is hydrogen, C₁-C₄-alkyl, C₇-C₈-aralkyl, or together with R⁵ is an optionally hydroxyl-substituted ethylene group, R⁵ = hydrogen and PG is C₁-C₅-acyl, C₁-C₅-alkyloxycarbonyl or C₃-C₅-alkenyloxycarbonyl.

8. Compounds according to Claim 7, in which R⁴ is hydrogen, methyl, isopropyl, isobutyl, benzyl, or together with R⁵ is an ethylene group or hydroxyethylene group, R⁵ = hydrogen and PG is acetyl, propionyl, butyryl, isopropionyl, isobutyryl, methoxycarbonyl, ethoxycarbonyl, isopropoxycarbonyl, tert-butoxycarbonyl or allyloxycarbonyl.

9. Compounds according to Claim 1 of the formula 1a 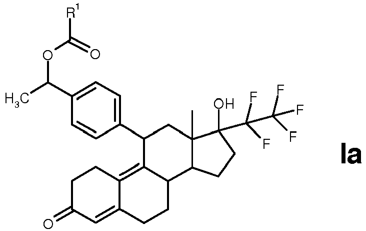 in which
R¹ is C₁-C₁₀-alkyl, (CH₂)ₙ-Y where n = 1-10 or CHR⁴NR⁵PG,
R⁴, R⁵ are each independently hydrogen, C₁-C₁₀-alkyl, C₇-C₁₀-aralkyl, or together are a (CH₂)ₘ- where m = 3 or 4 or a CH₂CHOHCH₂-group,
PG is hydrogen or an amino protecting group
Y is aryl or heteroaryl,
and the salts, solvates or solvates of the salts thereof.

10. Compounds according to Claim 9, in which R¹ is methyl, isopropyl, isobutyl or ^{tert}butyl.

11. Compounds according to Claim 9, in which R¹ is (CH₂)₂-Y and Y is 2-methylimidazol-1-yl or thiazol-1-yl.

12. Compounds according to Claim 9, in which R¹ is CHR⁴NR⁵PG and R⁴ is methyl or isopropyl, R⁵ is hydrogen or R⁴ together with R⁵ is a -(CH₂)₃- group and PG is hydrogen, -COOC(CH₃)₃ or -COOCH₂CH=CH₂.

13. The compounds according to any of the preceding claims, namely
acetic acid (RS)-1-[4-((8S,11R,13S,14S,17S)-17-hydroxy-13-methyl-3-oxo-17-pentafluoroethyl-2,3,6,7,8,11,12,13,14,15,16, 17-dodecahydro-1*H-*cyclopenta[*a*]phenanthren-11-yl)phenyl]ethyl ester (Example 1)
isobutyric acid (RS)-1-[4-((8S,11R,13S,14S,17S)-17-hydroxy-13-methyl-3-oxo-17-pentafluoroethyl-2,3,6,7,8,11,12,13,14,15,16, 17-dodecahydro-1*H-*cyclopenta[*a*]phenanthren-11-yl)phenyl]ethyl ester (Example 2)
3-methylbutyric acid (RS)-1-[4-((8S,11R, 13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]ethyl ester (Example 3)
3,3-dimethylbutyric acid (RS)-1-[4-((8S, 11R,13S,14S,17S)-17-hydroxy-13-methyl-3-oxo-17-pentafluoroethyl-2,3,6,7,8,11,12, 13,14,15,16,17-dodecahydro-1*H*-cyclopenta[*a*]phenanthren-11-yl)phenyl]ethyl ester (Example 4)
3-(2-methylimidazol-1-yl)propionic acid (S)-1-[4-((8S,11R,13S,14S,17S)-17-hydroxy-13-methyl-3-oxo-17-pentafluoroethyl-2,3,6,7,8,11, 12,13,14,15,16 17-dodecahydro-1*H*-cyclopenta[*a*]phenanthren-11-yl)phenyl]ethyl ester (Example 14)
3-(2-methylimidazol-1-yl)propionic acid (R)-1-[4-((8S,11R,13S,14S,17S)-17-hydroxy-13-methyl-3-oxo-17-pentafluoroethyl-2,3,6,7,8,11, 12,13,14,15,16 17-dodecahydro-1*H*-cyclopenta[*a*]phenanthren-11-yl)phenyl]ethyl ester (Example 14)
3-thiazol-2-ylpropionic acid (RS)-1-[4-((8S,11R,13S,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)phenyl]ethyl ester (Example 15)
(S)-2-*tert*-butoxycarbonylamino-3-methylbutyric acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl ester (Example 5)
(R)-2-*tert*-butoxycarbonylaminopropionic acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl ester (Example 6)
(S)-2-*tert*-butoxycarbonylaminopropionic acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl ester (Example 7)
(S)-2-aminopropionic acid (RS)-1-[4-((8S, 11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]ethyl ester (Example 8)
(S)-2-allyloxycarbonylaminopropionic acid (R)-1-[4-((8S,11R,13S,14S,17S)-17-hydroxy-13-methyl-3-oxo-17-pentafluoroethyl-2,3,6,7,8,11,12,13,14,15,16, 17-dodecahydro-1*H-*cyclopenta[*a*]phenanthren-11-yl)phenyl]ethyl ester (Example 9)
(S)-2-allyloxycarbonylaminopropionic acid (S)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl ester (Example 10)
(S)-pyrrolidine-1,2-dicarboxylic acid 1-tert-butyl ester 2-{(RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl} ester (Example 11)
(S)-pyrrolidine-2-carboxylic acid (RS)-1-[4-((8S,11R,13S,14S,17S)-17-hydroxy-13-methyl-3-oxo-17-pentafluoroethyl-2,3,6,7,8, 11,12,13,14,15,16,17-dodecahydro-1H-cyclopenta[a]phenanthren-11-yl)phenyl]ethyl ester (Example 12)
(S)-pyrrolidine-2-carboxylic acid (RS)-1-[4-((8S,11R,13S,14S,17S)-17-hydroxy-13-methyl-3-oxo-17-pentafluoroethyl-2,3,6,7,8, 11,12,13,14,15,16,17-dodecahydro-1H-cyclopenta[a]phenanthren-11-yl)phenyl]ethyl ester hydrochloride (Example 13)
(S)-2-allyloxycarbonylamino-3-methylbutyric acid (R)-1-[4-((8S,11R,13S,14S,17S)-17-hydroxy-13-methyl-3-oxo-17-pentafluoroethyl-2,3,6,7,8,11,12,13,14,15,16, 17-dodecahydro-1*H-*cyclopenta[*a*]phenanthren-11-yl)phenyl]ethyl ester (Example 16).

14. Compounds according to Claim 1, namely
acetic acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl ester (Example 1),
isobutyric acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*]phenanthren-11-yl)phenyl]ethyl ester (Example 2),
3-methylbutyric acid (RS)-1-[4-((8S,11R, 13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]ethyl ester (Example 3),
3,3-dimethylbutyric acid (RS)-1-[4-((8S, 11R,13S,14S,17S)-17-hydroxy-13-methyl-3-oxo-17-pentafluoroethyl-2,3,6,7,8,11,12,13,14,15,16,17-dodecahydro-1*H*-cyclopenta[*a*]phenanthren-11-yl)phenyl]ethyl ester (Example 4),
(S)-2-*tert*-butoxycarbonylamino-3-methylbutyric acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*] phenanthren-11-yl)phenyl]ethyl ester (Example 5),
(R)-2-*tert*-butoxycarbonylaminopropionic acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*] phenanthren-11-yl)phenyl]ethyl ester (Example 6),
(S)-2-*tert*-butoxycarbonylaminopropionic acid (RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*] phenanthren-11-yl)phenyl]ethyl ester (Example 7),
(S)-2-allyloxycarbonylaminopropionic acid (R)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*] phenanthren-11-yl)phenyl]ethyl ester (Example 9),
(S)-pyrrolidine-1,2-dicarboxylic acid 1-*tert*-butyl ester 2-{(RS)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[a] phenanthren-11-yl)phenyl]ethyl} ester (Example 11),
3-(2-methylimidazol-1-yl)propionic acid (R)-1-[4-((8S,11R,13S,14S,17S)-17-hydroxy-13-methyl-3-oxo-17-pentafluoroethyl-2,3,6,7,8,11,12,13,14,15,16 17-dodecahydro-1*H*-cyclopenta[*a*] phenanthren-11-yl)phenyl]ethyl ester (Example 14A),
3-thiazol-2-ylpropionic acid (RS)-1-[4-((8S,11R,13S,14S,17S)-17-hydroxy-13-methyl-3-oxo-17-pentafluoroethyl-2,3,6,7,8,11,12,13,14, 15,16,17-dodecahydro-1*H*-cyclopenta[*a*]phenanthren-11-yl)phenyl]ethyl ester (Example 15),
(S)-2-allyloxycarbonylamino-3-methylbutyric acid (R)-1-[4-((8S,11R,13S,14S,17S)-17-hydroxy-13-methyl-3-oxo-17-pentafluoroethyl-2,3,6,7,8,11,12,13,14,15,16,17-dodecahydro-1*H-*cyclopenta[*a*] phenanthren-11-yl)phenyl]ethyl ester (Example 16)
with a half-life in human plasma, determined in vitro, of greater than 100 hours.

15. Compound as defined in any of Claims 1 to 14 for treatment and/or prophylaxis of disorders.

16. Compound as defined in any of Claims 1 to 14 for treatment and/or prophylaxis of fibroids of the uterus (myomas, uterine leiomyomas), endometriosis, heavy menstrual bleeds, meningiomas, hormone-dependent breast cancers and complaints associated with the menopause.

17. Compound as defined in any of Claims 1 to 14 for fertility control and emergency contraception.

18. Use of a compound as defined in any of Claims 1 to 14 for production of a medicament for treatment and/or prophylaxis of disorders.

19. Use of a compound as defined in any of Claims 1 to 14 for production of a medicament for treatment and/or prophylaxis of fibroids of the uterus (myomas, uterine leiomyomas), endometriosis, heavy menstrual bleeds, meningiomas, hormone-dependent breast cancers and complaints associated with the menopause.

20. Use of a compound as defined in any of Claims 1 to 14 for production of a composition for fertility control and emergency contraception.

21. Medicament comprising a compound as defined in any of Claims 1 to 14 in combination with one or more inert, non-toxic, pharmaceutically suitable excipients.

22. Medicament comprising a compound as defined in any of Claims 1 to 14 in combination with one or more further active ingredients.

23. Medicament according to Claim 22, wherein the further active ingredient is selected from the group of SERMs, SERDs, antioestrogens, aromatase inhibitors, kinase inhibitors, angiogenesis inhibitors and/or cytostatics.

24. Medicament according to Claim 22, wherein the further active ingredient is selected from the group of the gestagens or gestagen/oestrogen combinations.

25. Medicament according to any of Claims 21 to 24 for treatment and prophylaxis of fibroids of the uterus (myomas, uterine leiomyomas), endometriosis, heavy menstrual bleeds, meningiomas, hormone-dependent breast cancers and complaints associated with the menopause.

## Revendications

1. Composé de formule I 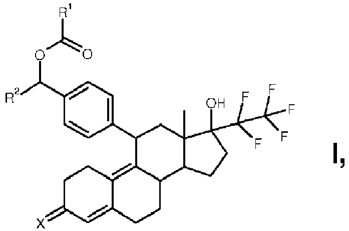 dans laquelle
X signifie oxygène, un groupe NOR³ ou =NNHSO₂R³
R¹ signifie C₁-C₁₀-alkyle, (CH₂)ₙ-Y ou CHR⁴NR⁵PG,
R² signifie hydrogène ou C₁-C₁₀-alkyle,
R³ signifie hydrogène, C₁-C₁₀-alkyle, aryle ou C₇-C₂₀-aralkyle,
n vaut 1 à 10,
Y signifie hydrogène, aryle ou hétéroaryle,
R⁴, R⁵ signifient, indépendamment l'un de l'autre, hydrogène, C₁-C₁₀-alkyle, C₇-C₁₀-aralkyle, ensemble un groupe (CH₂)ₘ- ou CH₂CHOHCH₂,
m vaut 3 ou 4,
PG signifie hydrogène ou un groupe de protection de la fonction amino,
et ses sels, ses solvates ou les solvates des sels, y compris toutes les modifications cristallines, les α-cyclodextrine-clathrates, les β-cyclodextrine-clathrates ou les γ-cyclodextrine-clathrates ainsi que les composés encapsulés par des liposomes, dans laquelle
aryle représente un radical carbocyclique monocyclique à tricyclique, aromatique, substitué ou non substitué, qui peut être monosubstitué ou polysubstitué par halogène (F, Cl, Br, I), OH, O-alkyle, CO₂H, CO₂-alkyle, NH₂, NH(C₁-C₁₀-alkyle), N(C₁-C₁₀-alkyle)₂, en particulier N(CH₃)₂, NO₂, N₃, CN, C₁-C₁₀-alkyle, C₂-C₁₀-alkylène, C₂-C₁₀-alcynyle, C₁-C₁₀-perfluoroalkyle, C₁-C₁₀-acyle, C₁-C₁₀-acyloxy,
hétéroaryle représente un radical monocyclique ou bicyclique, aromatique, comprenant généralement 5 à 10, et jusqu'à 5 hétéroatomes de la série S, O et N, par exemple, qui peut être monosubstitué par C₁-C₄-alkyle, aralkyle représente des groupes aralkyle et peut être monosubstitué ou polysubstitué par halogène, OH, 0-alkyle, CO₂H, CO₂-alkyle, NH₂, NH(C₁-C₁₀-alkyle), N(C₁-C₁₀-alkyle)₂, NO₂, N₃, CN, C₁-C₂₀-alkyle, C₁-C₁₀-perfluoroalkyle, C₁-C₂₀-acyle, C₁-C₂₀-acyloxy.

2. Composés selon la revendication 1, dans lesquels R¹ signifie méthyle, isopropyle, isobutyle ou néopentyle.

3. Composés selon la revendication 1, dans lesquels R¹ signifie (CH₂)ₙ-Y avec n = 1-10 et Y représente un radical aromatique, monocyclique ou bicyclique comprenant 5 à 10 atomes de cycle et jusqu'à 5 hétéroatomes de la série S, O et N.

4. Composés selon la revendication 3, dans lesquels n = 1-5 et Y représente un radical aromatique, monocyclique ou bicyclique comprenant 5 à 9 atomes de cycle et jusqu'à 4 hétéroatomes de la série S, O et N.

5. Composés selon la revendication 4, dans lesquels n = 1-3 et Y = imidazolyle, thiazolyle ou pyridyle.

6. Composés selon la revendication 1, dans lesquels R¹ représente un radical d'acide aminé CHR⁴NR⁵PG et R⁴ signifie hydrogène, C₁-C₅-alkyle, C₇-C₁₀-aralkyle ou, ensemble avec R⁵, un groupe éthylène ou propylène le cas échéant substitué par hydroxyle, R⁵ signifie hydrogène et PG signifie C₁-C₅-acyle, C₁-C₅-alkyloxycarbonyle ou C₃-C₅-alkényloxycarbonyle.

7. Composés selon la revendication 6, dans lesquels R⁴ signifie hydrogène, C₁-C₄-alkyle, C₇-C₈-aralkyle ou, ensemble avec R⁵, un groupe éthylène le cas échéant substitué par hydroxyle, R⁵ = hydrogène et PG signifie C₁-C₅-acyle, C₁-C₅-alkyloxycarbonyle ou C₃-C₅-alkényloxycarbonyle.

8. Composés selon la revendication 7, dans lesquels R⁴ signifie hydrogène, méthyle, isopropyle, isobutyle, benzyle ou, ensemble avec R⁵, un groupe éthylène ou hydroxyéthylène, R⁵ = hydrogène et PG signifie acétyle, propionyle, butyryle, isopropionyle, isobutyryle, méthoxycarbonyle, éthoxycarbonyle, isopropoxycarbonyle, tert-butoxycarbonyle ou allyloxycarbonyle.

9. Composés selon la revendication 1 de formule 1a : 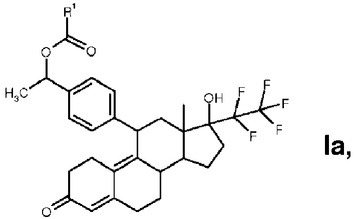 dans laquelle
R¹ signifie C₁-C₁₀-alkyle, (CH₂)ₙ-Y avec n = 1-10 ou CHR⁴NR⁵PG,
R⁴, R⁵ signifient, indépendamment l'un de l'autre, hydrogène, C₁-C₁₀-alkyle, C₇-C₁₀-aralkyle, ensemble un groupe (CH₂)m- avec m = 3 ou 4 ou CH₂CHOHCH₂,
PG signifie hydrogène ou un groupe de protection de la fonction amino,
Y signifie aryle ou hétéroaryle,
ainsi que ses sels, ses solvates ou les solvates des sels.

10. Composés selon la revendication 9, dans lesquels R¹ signifie méthyle, isopropyle, isobutyle ou tert-butyle.

11. Composés selon la revendication 9, dans lesquels R¹ signifie (CH₂)₂-Y et Y signifie 2-méthylimidazol-1-yle ou thiazol-1-yle.

12. Composés selon la revendication 9, dans lesquels R¹ signifie CHR⁴NR⁵PG et R⁴ signifie méthyle ou isopropyle, R⁵ signifie hydrogène ou R⁴ signifie, ensemble avec R⁵, un groupe -(CH₂)₃- et PG signifie hydrogène, -COOC(CH₃)₃ ou -COOCH₂CH=CH₂.

13. Composés selon l'une quelconque des revendications précédentes, à savoir
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide acétique (exemple 1)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide isobutyrique (exemple 2)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-méthylbutyrique (exemple 3)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3,3-diméthylbutyrique (exemple 4)
ester (S)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-(2-méthylimidazol-1-yl)-propionique (exemple 14)
ester (R)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-(2-méthylimidazol-1-yl)-propionique (exemple 14)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-thiazol-2-yl-propionique (exemple 15)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-tert-butoxycarbonylamino-3-méthylbutyrique (exemple 5)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (R)-2-tert-butoxycarbonylaminopropionique (exemple 6)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-tert-butoxycarbonylaminopropionique (exemple 7)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-aminopropionique (exemple 8)
ester (R)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-allyloxycarbonylaminopropionique (exemple 9)
ester (S)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-allyloxycarbonylaminopropionique (exemple 10)
ester 2-{(RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique} de l'ester 1-tert-butylique de l'acide (S)-pyrrolidine-1,2-dicarboxylique (exemple 11)
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-pyrrolidine-2-carboxylique (exemple 12)
chlorhydrate de l'ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-pyrrolidine-2-carboxylique (exemple 13)
ester (R)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-allyloxycarbonylamino-3-méthylbutyrique (exemple 16).

14. Composés selon la revendication 1, à savoir
ester (RS)-1-[4-((8S,11R,13S,14S,17S)-17-hydroxy-13-méthyl-3-oxo-17-pentafluoréthyl-2,3,6,7,8,11,12,13,14,15,16,17-dodécahydro-1H-cyclopenta[a]phénanthrén-11-yl)-phényl]-éthylique de l'acide acétique (exemple 1),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide isobutyrique (exemple 2),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-méthylbutyrique (exemple 3),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3,3-diméthylbutyrique (exemple 4),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-tert-butoxycarbonylamino-3-méthylbutyrique (exemple 5),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (R)-2-tert-butoxycarbonylaminopropionique (exemple 6),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-tert-butoxycarbonylaminopropionique (exemple 7),
ester (R)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-allyloxycarbonylaminopropionique (exemple 9),
ester 2-{(RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique} de l'ester 1-tert-butylique de l'acide (S)-pyrrolidine-1,2-dicarboxylique (exemple 11),
ester (S)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-(2-méthylimidazol-1-yl)-propionique (exemple 14A),
ester (RS)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide 3-thiazol-2-yl-propionique (exemple 15)
ester (R)-1-[4-((8S,11R,13S,14S,17S)-17-hydroxy-13-méthyl-3-oxo-17-pentafluoroéthyl-2,3,6,7,8,11,12,13,14,15,16,17-dodécahydro-1H-cyclopenta[a]phénanthrén-11-yl)-phényl]-éthylique de l'acide (S)-2-allyloxycarbonylamino-3-méthylbutyrique (exemple 16)
présentant une durée de demi-vie dans le plasma humain, déterminée in vitro, supérieure à 100 heures.

15. Composé tel que défini dans l'une quelconque des revendications 1 à 14, destiné au traitement et/ou à la prophylaxie de maladies.

16. Composé tel que défini dans l'une quelconque des revendications 1 à 14, destiné au traitement et/ou à la prophylaxie de fibromes utérins (myomes, léiomyomes utérins), de l'endométriose, de saignements menstruels importants, de méningiomes, de carcinomes mammaires dépendant des hormones et de troubles associés à la ménopause.

17. Composé tel que défini dans l'une quelconque des revendications 1 à 14, destiné au contrôle de la fertilité et à la contraception d'urgence.

18. Utilisation d'un composé tel que défini dans l'une quelconque des revendications 1 à 14, pour la préparation d'un médicament destiné au traitement et/ou à la prophylaxie de maladies.

19. Utilisation d'un composé tel que défini dans l'une quelconque des revendications 1 à 14, pour la préparation d'un médicament destiné au traitement et/ou à la prophylaxie de fibromes utérins (myomes, léiomyomes utérins), de l'endométriose, de saignements menstruels importants, de méningiomes, de carcinomes mammaires dépendant des hormones et de troubles associés à la ménopause.

20. Utilisation d'un composé tel que défini dans l'une quelconque des revendications 1 à 14, pour la préparation d'un agent destiné au contrôle de la fertilité et à la contraception d'urgence.

21. Médicament contenant un composé, tel que défini dans l'une quelconque des revendications 1 à 14, en combinaison avec un ou plusieurs adjuvants inertes, non toxiques, pharmaceutiquement appropriés.

22. Médicament contenant un composé tel que défini dans l'une quelconque des revendications 1 à 14 en combinaison avec une ou plusieurs autres substances actives.

23. Médicament selon la revendication 22, l'autre substance active étant choisie dans le groupe des SERM, des SERD, des anti-estrogènes, des inhibiteurs des aromatases, des inhibiteurs des kinases, des inhibiteurs de l'angiogenèse et/ou des cytostatiques.

24. Médicament selon la revendication 22, l'autre substance active étant choisie dans le groupe des gestagènes ou des combinaisons gestagène/estrogène.

25. Médicament selon l'une quelconque des revendications 21 à 24, destiné au traitement et à la prophylaxie de fibromes utérins (myomes, léiomyomes utérins), de l'endométriose, de saignements menstruels importants, de méningiomes, de carcinomes mammaires dépendant des hormones et de troubles associés à la ménopause.
